# EUROPÄISCHE PATENTANMELDUNG

(11) **EP 1 626 095 A1**
(43) Veröffentlichungstag der Anmeldung: **15.02.2006**
(21) Anmeldenummer: 04019090.2
(22) Anmeldetag: 11.08.2004
(51) Int. Cl.: C12Q 1/52, C12Q 1/56

(54) **Fluoreszenzbasierte kinetische Bestimmung der Aktivität von Transglutaminasen**

(71) Anmelder: N-Zyme BioTec GmbH, 64295 Darmstadt (DE)
(72) Erfinder: Pasternack, Ralf, 64347 Griesheim (DE); Oertel, Kai, 55122 Mainz (DE)
(74) Vertreter: Grünecker, Kinkeldey, Stockmair & Schwanhäusser Anwaltssozietät

(57) **Zusammenfassung**

Verfahren zur fluoreszenzbasierten kinetischen Messung von Transglutaminase-Aktivität, dadurch gekennzeichnet, dass die Transglutaminase mit einem modifizierten Peptidsubstrat, bei dem ein Fluorophor direkt an den N-Terminus des Peptids gebunden ist und ein Glutaminsäurerest in 5-Position einen Fluoreszenzquencher trägt, welcher über einen Spacer, der eine Aminfunktion enthält, unter Ausbildung einer Amidbindung an die 5-Position gebunden ist, umgesetzt wird, so dass durch die Aktivität der Transglutaminase der Fluoreszenzquencher abgespalten wird, und die resultierende Fluoreszenz des Restmoleküls gemessen wird.

## Beschreibung

Die vorliegende Erfindung betrifft ein fluoreszenzbasiertes Verfahren zur qualitativen und quantitativen Bestimmung der Aktivität von Transglutaminasen unter Verwendung modifizierter Peptidsubstrate.

Im menschlichen Körper wurden bis heute neun verschiedene Transglutaminasen identifiziert. Transglutaminasen spielen eine immer größer werdende Rolle in der medizinischen Diagnostik, nachdem eine wachsende Anzahl an Krankheiten mit Transglutaminasen in Verbindung gebracht wurden (Gentile, V., et al., Current Drug Targets - CNS & Neurological Disorders, 3, 2004, 69-74; Kim, S.Y., et al., Neurochem Int. 2002, 85-103, Cooper A. J., Neurochem Int., 2002, 1-5). Bereits heute wird in der klinischen Routinediagnostik die Messung des Blutgerinnungsfaktors XIII (FXIII) durchgeführt, da mit einem Mangel dieser Transglutaminase ernsthafte Erkrankungen verbunden sind *(Klinische Aspekte des Faktor-XIII-Mangels,* Karger Verlag, 1999, Hrsg.: R. Egbring, R.Seitz, G. Wozniak).

Obwohl eine Vielzahl an Aktivitätstests für Transglutaminasen bekannt ist, existiert bis heute kein Verfahren, welches allgemein anwendbar, einfach und schnell in der Durchführung ist, eine hohe Sensitivität besitzt und gleichzeitig eine hohe Selektivität zwischen den verschiedenen Transglutaminasen gewährleistet.

Im Wesentlichen werden zur quantitativen Messung die folgenden Methoden benutzt: Einbau eines markierten Amines in ein Protein und direkte oder indirekte Quantifizierung des Markers, z.B. der Einbau von Dansylcadaverin in N,N-Dimethylcasein (US 4,601,977, Lorand L. et al., *J. Clin. Invest.*,48, 1969, 1054-64) bzw. von Biotinylcadaverin in N,N-Dimethylcasein (US 5,015,588; Lee K.N. et al., *Clin. Chem.,* 34, 1988, 906-10; Song Y.C. et al., *Anal. Biochem.,* 223, 1994, 88-92), sowie Radioisotopen (Dviansky A. et al., *Br. J. Haematol*. 18, 1970, 399-410; Lorand L. et al., *Anal. Biochem.,* 50, 1972, 623-31). Eine andere quantitative Methode misst die Menge an gebildetem Ammoniak, die durch Hydrolyse bzw. Aminolyse eines zugesetzten synthetischen Peptids entsteht (US 5,204,240, Clin. Chem., Vol. 31, Nr. 1, 1985, 35-40).

Andere Methoden sind speziell für eine bestimmte Transglutaminase bekannt, wie zum Beispiel die Messung des Vernetzungsgrades von Fibrin zur Bestimmung der Aktivität des FXIII (US 5,508,202 und US 6,406,874; siehe auch Karges, R., Clemens R., *Behring Inst. Mitt.* 82, 1988, 43-58). Neben diesen Testverfahren sind in der wissenschaftlichen Literatur eine Vielzahl von Verfahren zur Messung von Transglutaminase-Aktivität bekannt. (Grossowicz, *J. Biol. Chem*. 187, 1950; Lorand L., *Anal. Biochem.,* 1971, 44, 221-231; Slaughter et al., *Anal Biochem.* 1992, 205(1):166-71; Aeschliman et al., *J. Biol. Chem.* 1995; 270(40):23415-20; 111-125; Lorand L., *J. Biol. Chem*. 1997, 10311-10317; Pasternack R. et al., *Anal Biochem.* 1997;249(1):54-60; Lorand L., *Exp- Eye Res.* 1998, 66, 531-536; Keillor J.W. et al., *Anal Biochem.* 1999 274(1):141-4; Keillor J.W. et al., *Anal Biochem.* 2000, 285(1):16-20; Furutani et al., *J Histochem* Cytochem. 2001 Feb;49(2):247-58; Jeitner T.M., *Anal Biochem.* 2001;292(2):198-206; Fesus L. et al., *Anal Biochem.* 2002, 311(2):187-90; Keillor J.W., *Biochemistry.* 2003;42(39):11504-13)

Gemeinsam ist diesen Verfahren, dass sie zur Beantwortung bestimmter wissenschaftlicher Fragen in der Grundlagenforschung geeignet sind. In der Handhabung und Durchführung sind sie jedoch zu zeitaufwändig und können zudem nicht zwischen verschiedenen Transglutaminasen unterscheiden. Auch die Genauigkeit der Messungen, inbesondere bei geringen Transglutaminaseaktivitäten, ist häufig nicht ausreichend. Gerade geringe Aktivitäten von Transglutaminasen sind aber in der medizinischen Diagnostik von entscheidender Bedeutung, da solche Mangelzustände (< 5 % des normalen physiologischen Levels), genetische Defekte und Polymorphismen erhebliche Relevanz für betroffene Patienten besitzen.

Parameswaran und Mitarbeiter beschreiben in einer Veröffentlichung die kinetische Aktivitätsmessung über die Hydrolyse von fluoreszenzmarkierten Peptiden, in denen das eigentliche Substratglutamin durch eine in 5-Position mit einer fluoreszenzquenchenden Molekülgruppe (2,4-Dinitrophenylcadaverin) substituierten Glutaminsäure ersetzt wird (Parameswaran et. al., *J. Biol. Chem*., 1997, 10311-17). Als Fluorophor finden N-[5-(Dimethylamino)-1-naphtalin-sulfonsäure]-ε-aminohexansäure bzw. N-(2-Aminobenzoyl)-ε-aminohexansäure Verwendung. Gemessen wird die durch Hydrolyse der Seitenkette und damit der räumlichen Entfernung des Fluoreszenzquenchers aus dem fluoreszierenden Peptid wachsende Fluoreszenz im Ansatz. (Zur Theorie des zugrunde liegenden Förster-Energie-Transfers siehe z.B. Szöllosi J., *Cytometry,* 34, 1998, 159-179). Die steigende Fluoreszenz kann als Maß für die Aktivität der Transglutaminase detektiert werden. In dieser Veröffentlichung werden ausschließlich solche Moleküle zum Einsatz gebracht, die den Fluorophor über einen Alkanoylspacer (hier 6-Aminohexansäure) am N-terminalen Ende der Peptidsequenz tragen. Als Peptidsequenz legen die Autoren ausschließlich Gln-Gln-Ile-Val zugrunde, wobei das N-terminale Glutamin in oben genannter Weise modifiziert wird.

Die Menge an Transglutaminase, die eingesetzt werden muss, um einen detektierbaren Anstieg der Fluoreszenz zu erreichen, liegt in diesem System jedoch z.B. für FXIIIₐ mit 100 mg/Liter wesentlich über den Konzentrationen, die physiologisch relevant sind (ca. 10 bis 15 mg/Liter).

In Anbetracht der geschilderten Nachteile des Standes der Technik besteht die Aufgabe der vorliegenden Erfindung in der Bereitstellung eines neuen selektiven Verfahrens zur schnellen, einfachen und und vor allem hochsensitiven kinetischen Messung von Transglutaminase-Aktivitäten, das auch für klinische Anwendungen geeignet ist.

Diese Aufgabe wird erfindungsgemäß gelöst durch ein fluoreszenzbasiertes Verfahren zur Bestimmung der Aktivität von Transglutaminasen unter Verwendung modifizierter Peptidsubstrate gemäß Anspruch 1.

Dabei werden modifizierte Peptide eingesetzt, die neben einem Fluorophor, das direkt an die N-terminale Aminogruppe des Peptids gebunden ist, eine Molekülgruppe in der 5-Position eines Glutaminsäurerestes tragen, welche die Fluoreszenz des Fluorophors weitgehend unterdrückt (Fluoreszenzquencher). Gemessen wird der Anstieg der Fluoreszenz durch hydrolytische oder aminolytische Abspaltung des Fluoreszenzquenchers durch die Aktivität der Transglutaminase.

Bei der Entwicklung des erfindungsgemäßen Verfahrens wurde überraschenderweise festgestellt, dass die molekulare Struktur sowie die Art der Anbindung des Fluorophors einen entscheidenden Einfluss auf die Substrateigenschaften des Peptidsubstrats und die Signalintensität bei der Aktivitätsmessung haben. Durch direkte Bindung von z.B. 2-Aminobenzoesäure (Abz) oder strukturell ähnlicher Verbindungen an den N-Terminus einer geeigneten Peptidsequenz werden Peptide erhalten, die, abhängig von der verwendeten Peptidsequenz, von verschiedenen Transglutaminasen sehr gut als Substrat erkannt werden. Die notwendige Konzentration an Substratpeptid ist entsprechend niedrig und liegt in der Regel im Bereich von 10-100 µM. Die guten Substrateigenschaften dieser fluoreszenzmodifizierten Peptide ermöglichen auch die Messung sehr geringer Transglutaminase-Aktivitäten, wie sie unter physiologischen Bedingungen häufig vorkommen, und die bisher nicht möglich waren.

Ein weiterer Vorteil des erfindungsgemäßen Verfahrens gegenüber dem Stand der Technik sind neben der Empfindlichkeit der Messung die Tatsache, dass damit eine diskriminierende Messung verschiedener Transglutaminasen möglich ist. Das bedeutet, dass ein Substrat, welches hinsichtlich der verwendeten Peptidsequenz für eine bestimmte Transglutaminase geeignet ist, von einer anderen Transglutaminase nicht oder nur sehr geringfügig umgesetzt wird. Sind beide Transglutaminasen zum Beispiel in einem Zellhomogenisat vergesellschaftet, so kann ihre Aktivität unabhängig voneinander gemessen werden.

Als Fluorophor kommen grundsätzlich alle Fluorophore in Frage, welche die gewünschte hohe Sensitivität des erfindungsgemäßen Verfahrens gewährleisten. Vorzugsweise handelt es sich dabei um am Benzolring substituierte Benzoesäurederivate, bevorzugter um Benzoesäurederivate, die an der 2-Position einen Substituenten tragen, besonders bevorzugt sind 2-Aminobenzoesäure und N-Methyl-2-aminobenzoesäure. "Direkte Bindung des Fluorophors" bedeutet im Kontext der vorliegenden Erfindung, dass sich keine Spacergruppierung zwischen dem Fluorophor und dem N-Terminus des Peptids befindet. So besteht z.B. im Falle der 2-Aminobenzoesäure eine direkte Amidbindung zwischen der Carboxylgruppe der Benzoesäure und der Aminofunktion des Peptids.

Der Fluoreszenzquencher muss hinsichtlich der spektralen Eigenschaften auf das Fluorophor abgestimmt sein und zueinander passende Paare von Fluorophor und Fluoreszenzquencher sind seit langem bekannt und werden häufig verwendet. Geeignete Partner für die erfindungsgemäß besonders bevorzugte 2-Aminobenzoylgruppierung sind Fluoreszenzquencher, die z.B. eine 2,4-Dinitrophenol- oder 3-Nitrotyrosingruppe enthalten bzw. daraus bestehen (vgl. z.B. Juliano, M.A., et al., J. *Pept.Res*., 53, 1999, 109-19).

Als Peptidsubstrat werden niedermolekulare Peptide eingesetzt, in denen das Glutamin, welches als Substrat-Aminosäure für die jeweilige Transglutaminase dient, durch eine Glutaminsäure ersetzt wird, die einen Fluoreszenzquencher trägt, welcher über einen Spacer, der eine Aminfunktion enthält, unter Ausbildung einer Amidbindung an die 5-Position der Glutaminsäure gebunden ist. Vorzugsweise ist dieser Spacer ein Alkylaminspacer, der in der Regel 1 bis 6, vorzugsweise 5, Methylengruppen zwischen Fluoreszenzquencher und Glutaminsäure enthält. Die zweite funktionelle Gruppe des Spacers zur Anbindung des Fluoreszenzquenchers kann in Abhängigkeit von der Molekülstruktur des speziell eingesetzten Fluoreszenzquenchers variieren. Insbesondere für Fluoreszenzquencher, die eine 2,4-Dinitrophenol- oder 3-Nitrotyrosingruppe enthalten, werden vorzugsweise 1,n-Alkydiaminspacer, besonders bevorzugt 1,5-Pentandiamin (Cadaverin, abgekürzt CAD) verwendet. Vorzugsweise werden als Substratpeptide solche Peptide eingesetzt, die selektiv für die zu messende Transglutaminase sind.

In einer bevorzugten Ausführungsform wird das Peptidsubstrat durch die Formel I repräsentiert 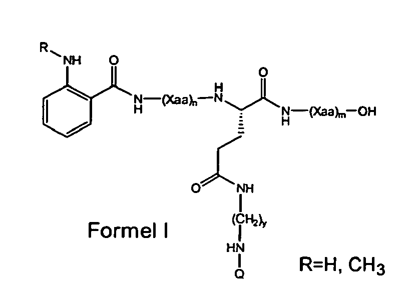
worin Xaa ein beliebige Aminosäure oder ein Aminosäurederivat davon ist,
n und und m unabhängig voneinander 0 bis 10 sind,
y 1 bis 6, vorzugsweise 5, ist,
Q ein Fluoreszenzquencher ist,
R H oder CH₃ ist.

Die neben dem modifizierten Glutaminsäurerest vorhandenen Aminosäuren des Peptidsubstrates, Xaa in der Formel I, können eine beliebige Aminosäure, vorzugsweise eine natürliche Aminosäure, oder ein die Substrateigenschaften nicht wesentlich beeinträchtigendes Derivat davon sein.

Geeignete, jedoch nicht beschränkende Beispiele für erfindungsgemäße selektive Peptidsubstrate sind Abz-Asn-Glu((CH₂)_{y}-NH-Q)-Glu-Gln-Val-Ser-Pro-Leu-Thr-Leu-Leu-Lys und Abz-Pyr-Ala-Glu((CH₂)_{y}-NH-Q)-Gln-Ile-Val für FXIII sowie Abz-Ala-Pro-Glu((CH₂)_{y}-NH-Q)-Gln-Glu-Ala für humane Gewebetransglutaminase (Transglutaminase 2) und die entsprechenden N-Methyl-Abz-Derivate davon.

Das erfindungsgemäße Verfahren kann zur Aktivitätsmessung jeder bekannten Transglutaminase genutzt werden, denn alle Mitglieder der Enzymfamilie katalysieren grundsätzlich dieselbe Reaktion. Bevorzugt sind bakterielle Transglutaminasen und Transglutaminasen aus Vertebraten, bevorzugter Säuger-Transglutaminasen, besonders bevorzugt humane Transglutaminasen. Nicht beschränkende Beispiele geeigneter humaner Transglutaminasen sind Faktor XIII, humane Transglutaminase 2, TG3, TG4, TG5, TG6, TG7.

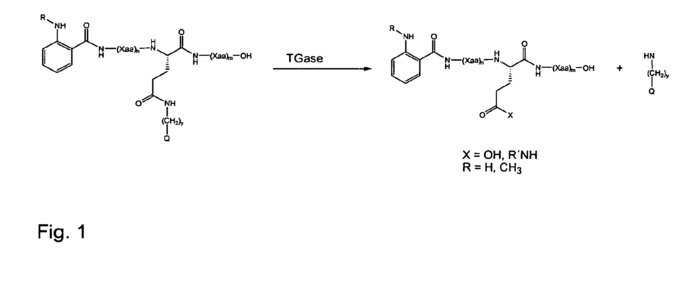

Eine typische Ausführungsform des erfindungemäßen Verfahrens ist die selektive Bestimmung von verschiedenen Transglutaminasen in Transglutaminase enthaltenden Proben, beispielsweise FXIII in Plasmen oder TG2 in Gewebeproben. Je nach Art der biologischen Probe können dabei zusätzliche Ingredenzien und Hilfsstoffe oder Vorbereitungsschritte von Vorteil sein. Zum Beispiel wird in unbehandelten Plasmen FXIII günstigerweise mit Thrombin aktiviert und ein Fibrinaggregationshemmer, vorzugsweise Gly-Pro-Arg-Pro-amid, zugesetzt. Bei anderen Proben könnte der Zusatz anderer Additive vorteilhaft oder notwendig sein. Dadurch wird jedoch die breite Einsetzbarkeit der erfindungsgemäßen modifizierten Peptidsubstrate, insbesondere der allgemeinen Formel 1, nicht eingeschränkt. In einer bevorzugten Ausführungsform wird dem Reaktionsansatz ein primäres Amin zugegeben, wodurch die Hydrolysereaktion zu einer Aminolysereaktion wird (X=RHN in Fig. 1). Dadurch kann die Signalstärke zusätzlich erhöht werden.

Einige geeignete, nicht beschränkende, Beispiele für solche Amine sind ein Glycinmethyl- oder-ethytester, Ethanolamin, Diaminoethan oder ein Derivat von 1,5-Diaminopentan.

Mit dem erfindungsgemäßen Verfahren kann eine Bestimmung der Transglutaminase-Aktivität in Proben schnell und sicher durchgeführt werden. Die Testausführung beinhaltet vorzugsweise folgende Schritte:
- Vorlegen der zu untersuchenden Probe;
- gegebenenfalls Zusatz von Aggregationshemmern oder anderen Hilfsstoffen, die gegebenenfalls in einer Pufferlösung vorliegen;
- gegebenenfalls Aktivierung einer Transglutaminase, z.B. FXIII durch Thrombin, und Aktivierung für einen gegebenen Zeitraum. Die Aktivierung kann z.B. in einer bereits calciumionen-haltigen Lösung durchgeführt werden oder den Zusatz von Calciumionen beinhalten.
- Zusatz von Substratlösung, die entweder in Pufferlösung oder in einem Gemisch aus Dimethylsulfoxid oder einen anderen geeignetem Lösungsmittel und Pufferlösung gelöst vorliegt. Vorzugsweise liegt das Substrat in einer Konzentration von 10 bis 100 µM im Endansatz vor.
- Messen der Fluoreszenzänderung.

Vorzugsweise sollten alle Lösungen vor Beginn der Messung auf eine Temperatur gebracht werden, bei der mit einer hohen Aktivität der Transglutaminase gerechnet werden kann. Eine für die meisten bekannten Transglutaminasen geeignete Temperatur beträgt etwa 37°C. Erforderlichenfalls kann die optimale Assay-Temperatur für eine bestimmte Transglutaminase von einem Fachmann durch Routineversuche festgestellt werden.

Die erfindungsgemäße Aktivitätsmessung kann sowohl als als Einzelmessung erfolgen als auch als parallele Messung mehrerer Proben in einer dazu geeigneten Vorrichtung für parallelisierte Meßverfahren. Solche Vorrichtungen sind im Stand der Technik an sich bekannt und können unschwer an das erfindungsgemäße Verfahren adaptiert werden. In einer speziellen Ausführungsform umfaßt die Vorichtung Mikrotiterplatten, in denen die Messungen vorgenommen werden können, und entsprechende Instrumente zur Messung der Fluoreszenz.

### FIGURENBESCHREIBUNG:

**Fig. 1** Transglutaminase-katalysierte Abspaltung eines Fluoreszenzquenchers von einem fluoreszenzmodifizierten Peptidsubstrat
**Fig. 2** Excitations- und Emissisionspektrum der nach dem Synthesebeispiel 1 erhaltenen Substratverbindung 2
**Fig. 3** Fluoreszenzänderung der Substratverbindung 2 mit der Zeit nach Umsetzung mit FXIII unter den in Beispiel 1 angebenen Reaktionsbedingungen
**Fig. 4** Fluoreszenzänderung der Substratverbindung 3 mit der Zeit nach Umsetzung mit humaner Transglutaminase 2 unter den in Beispiel 2 angegebenen Reaktionsbedingungen
**Fig. 5** Fluoreszenzänderung der Substratverbindung 3 mit der Zeit nach Umsetzung mit Meerschweinchenleber-Transglutaminase unter den in Beispiel 3 angegebenen Reaktionsbedingungen

### SYNTHESEBEISPIEL 1

### a) Herstellung von N-(2,4-Dinitrophenyl)-1,5-diaminopentan-Hydrochlorid

1,09 g N-(tert.-Butyloxycarbonyl)-1,5-diaminopentan (5,37 mmol) wird in 20 ml Ethanol vorgelegt und bei Raumtemperatur unter Argonatmosphäre nacheinander mit 4,92 ml N-Ethyldiisopropylamin (29 mmol) sowie einer Lösung von 1 g 2,4-Dinitrofluorbenzol (5,37 mmol) in 20 ml Benzol versetzt. Die orange Lösung wird bei Raumtemperatur über Nacht gerührt. Die Lösungsmittel werden im Vakuum entfernt. Der erhaltene Rückstand wird in 150 ml Ethylacetat aufgenommen, jeweils dreimal mit 50 ml 10%iger wässeriger Citronensäurelösung und Wasser gewaschen. Nach Trocknen der organischen Phase mit Na₂SO₄ wird das Lösungsmittel im Vakuum entfernt. Nach erneuter Aufnahme in 20 ml absolutem Dichlormethan und Zusatz von 5 ml einer 4 M Lösung HCl in Dioxan wird für eine Stunde bei Raumtemperatur gerührt. Nach Entfernung des Lösungsmittels im Vakuum erhält man das Produkt als gelbes Pulver.
Ausbeute: 1,94 g (94%)
ESI-MS: 269,0 [M+H]⁺

### b) Herstellung von N^{α}-9-Fluorenylmethoxycarbonyl-N^{δ}-[N-(2,4-dinitrophenyl)]-5-aminopentyl]-L-glutamin (1)

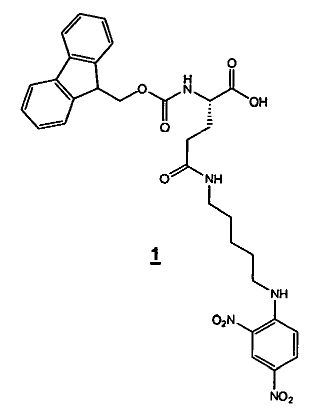

500 mg N^{α}-Fluorenylmethoxycarbonyl-L-glutaminsäure-1-tert.-butylester (1,175 mmol) werden in 10 ml DMF gelöst und mit 382 µl N-Ethyldiisopropylamin (2,24 mmol) versetzt. Unter Argonatmosphäre wird auf -20 °C gekühlt und 161 µl Isobutylchlorformiat (1,175 mmol) zugesetzt. Bei -20°C wird für 30 Minuten gerührt und anschließend eine Lösung aus 326 mg N-[N'-(2,4-Dinitrophenyl)]-1,5-diaminopentan-Hydrochlorid (1,07 mmol) in 5 ml DMF zugegeben. Unter Lichtausschluss wird über Nacht gerührt und währenddessen allmählich auf Raumtemperatur aufgetaut. Das Lösungsmittel wird im Vakuum entfernt und der gelbe, ölige Rückstand in 100 ml Ethylacetat aufgenommen. Es wird jeweils dreimal mit 20 ml 10 %iger wässeriger Citronensäurelösung und Wasser gewaschen. Nach Trocknen der organischen Phase mit Na₂SO₄ wird das Lösungsmittel im Vakuum entfernt. Das erhaltene gelbe Harz, wird in 10 ml absolutem Dichlormethan gelöst und mit 10 ml Trifluoressigsäure versetzt. Bei Raumtemperatur wird für eine Stunde gerührt und danach zur Trockne eingeengt. Die Reingung erfolgt durch Chromatographie an Kieselgel (Säule: 3,9 x 28 cm, Laufmittel: Dichlormethan/Methanol = 90/10, (v/v) R_{f} = 0,1).
Ausbeute: 630 mg (95 % d. Th.)
ESI-MS: 620,4 [M+H]⁺, 622,4 [M+Na]⁺

### c) Herstellung von 2-Aminobenzoyl-L-asparaginyl-N^{δ}-[N-(2,4-dinitrophenyl)1-5-aminopentyl]-L-glutaminyl-L-glutamyl-L-glutaminyl-L-valinyl-L-serinyl-L-prolinyl-L-leucinyl-L-threoninyl-L-leucinyl-L-leucin-L-lysin (2)

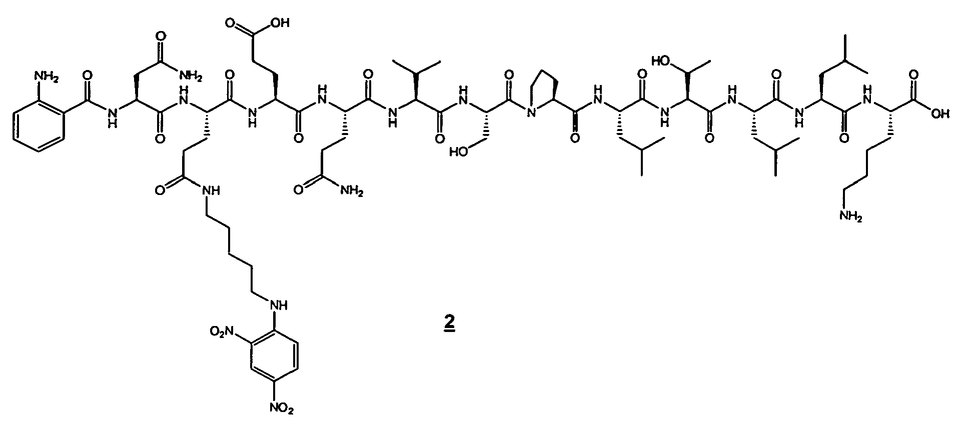

Die Titelverbindung wird nach Standardmethoden der Fmoc-Festphasenpeptidsynthese an einem Polystyrolträger mit der Chlortritylankergruppe dargestellt. Als elfte Aminosäure wird N^{α}-9-Fluorenylmethoxycarbonyl-N^{δ}-[N-(2,4-dinitrophenyl)]-5-aminopentyl]-L-glutamin (1) in die Peptidsequenz eingeführt. Diese modifizierte Aminosäure wird in üblicher Weise durch Aktivierung mit TBTU an das Decapeptid (EQVSPLTLLK) gebunden. Durch abschließende Umsetzung mit N^{α}-9-Fluorenylmethoxycarbonyl-N-β-trityl-L-asparagin wird die Peptidsequenz vervollständigt. Nach Entfernen der N-terminalen Schutzgruppe von dem Dodecapeptid wird 2-(tert.Butyloxycarbonylamino)benzoesäure ebenfalls durch Aktivierung mit TBTU an den N-Terminus des Peptides gebunden. Durch Behandlung des polymeren Trägers mit einer Mischung aus 95% Trifluoressigsäure / 2,5% Trisopropysilan / 2,5% Wasser wird die Titelverbindung als gelber Feststoff erhalten. Die Reinigung erfolgt durch präparative HPLC unter Umkehrphasenbedingungen (Säule: Synergie Max, 4µm, 80 Angström (Phenomenex), 250x21,2 mm, Laufmittel: A-Eluent = 0,1% Trifluoressigsäure in Wasser, B-Eluent = 0,1% Trifluoressigsäure in 9,9% Wasser und 90 % Acetonitril, Gradient: 5% B-Eluent für 15 Minuten. Dann in 95 Minuten auf 100% B-Eluent, Fluss: 8 ml/min. Rₜ = 65,2 min - 68,6 min).
ESI-MS: 1739,9 [M+H]⁺, 1761,9 [M+Na]⁺

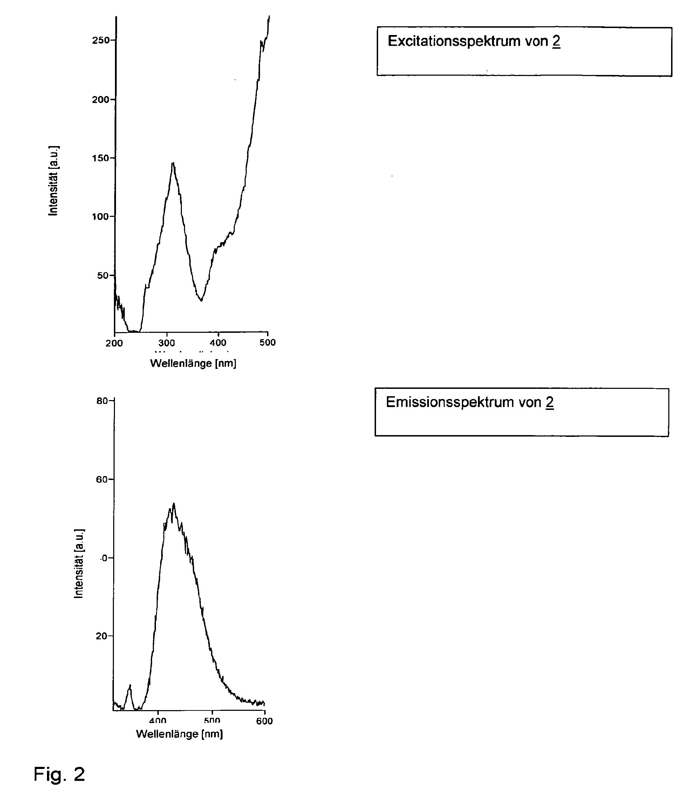

Die Excitations- und Emissionsspektren von 2 sind in Fig. 2 dargestellt.

### SYNTHESEBEISPIEL 2

### Herstellung von 2-Aminobenzoyl-L-alaninyl-L-Prolinyl-N^{δ}-[N-(2,4-dinitrophenyl)]-5-aminopentyl]-L-glutaminyl-L-glutaminyl-L-glutamyl-alanin (3)

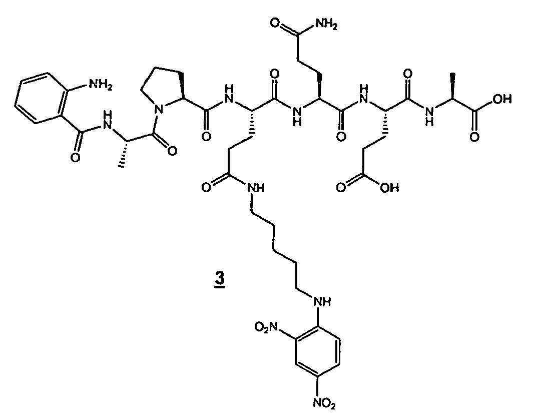

Die Darstellung erfolgt analog der unter Synthesebeispiel 1 angegebenen Weise unter Verwendung der für die obige Peptidsequenz erforderlichen Aminosäuren.
Die Reinigung erfolgt ebenfalls unter den gleichen Bedingungen.
Rₜ = 63-65 min
ESI-MS: 1014,3 [M+H]⁺, 1036,3 [M+Na]⁺

### BEISPIEL 1

### Kinetische Messung von FXIII in Plasmen

### Substratpeptid: Abz-Asn-Glu(CAD-DNP)-Glu-Gln-Val-Ser-Pro-Leu-Thr-Leu-Leu-Lys(2)

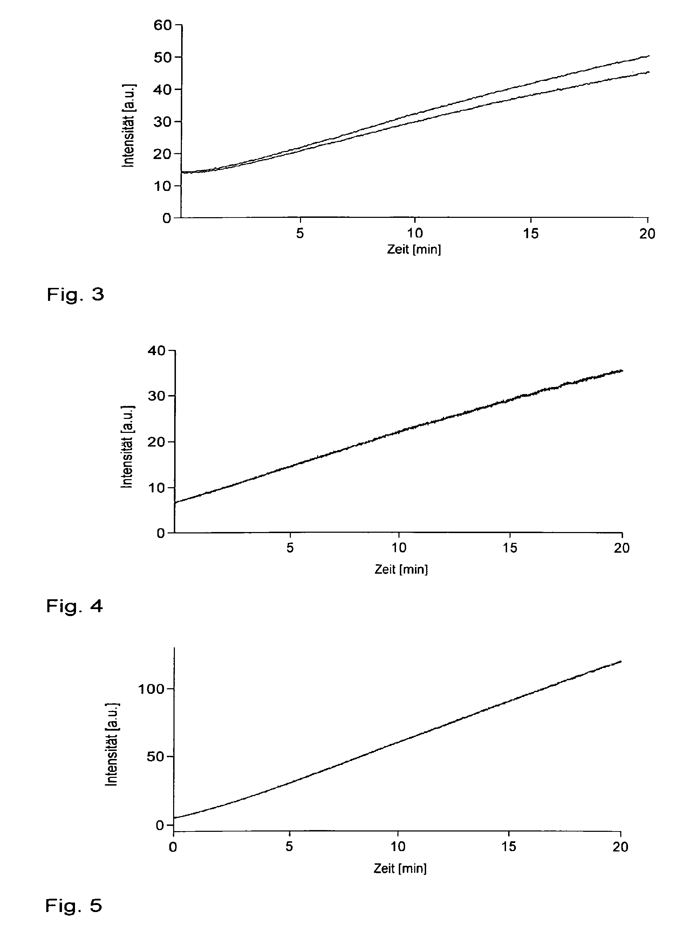

Die Substratsequenz Asn-Gln-Glu-Gln-Val-Ser-Pro-Leu-Thr-Leu-Leu-Lys ist einem natürlichen Substrat des FXIII entnommen und wurde bereits zur kinetischen Messungen von FXIII in der Literatur beschrieben (Kárpáti L. et al., Clin Chem., 46:12, 2000, 1946-55). Durch die erfindungsgemäße Modifikation dieses Peptides kann eine kinetische Messung in Plasmen durchgeführt werden (unterer Graph in Fig. 3). Die Messung wurde in diesem speziellen Fall wie folgt durchgeführt.

| | |
|---|---|
| Ansatzgröße: | 1 ml |
| Substratlösung: | 800 µl |
| Probenvolumen: | 100 µl Citratplasma |
| Thrombinlösung: | 100 µl |
| Ansetzen der Stammlösung: | 7 mg Substrat und 90 mg GPRP-NH₂ in 1 ml DMSO lösen und mit 7 ml Puffer (50 mM Tris-HCl (pH = 7,5), 10 mM CaCl₂, 100 mM NaCl, 0,1 % PEG₈₀₀₀) verdünnen. |
| Ansetzen der Substratlösung: | Stammlösung 1/10 (v/v) mit Puffer verdünnen. |
| Durchführung der Messung: | 800 µl Substratlösung mit 100 µl Plasma versetzen, Aktivierung durch Zusatz von 20 NIH Units Thrombin in 100 µl Puffer. Messung bei 37°C. Start der Messung nach 5 min. Exc.: 310 nm Em.: 427 nm, Messung für 10 min |

Führt man die Reaktion in Gegenwart von 5 mM Glycinethylester unter ansonsten identischen Bedingungen durch, wird das Signal zusätzlich verstärkt (oberer Graph in Fig. 3)

### BEISPIEL 2

### Kinetische Messung von gereinigter Transglutaminase 2 (Gewebetransglutaminase)

### Substratpeptid: Abz-Ala-Pro-Glu(CAD-DNP)-Gln-Glu-Ala (3)

Die Substratsequenz Ala-Pro-Gln-Gln-Glu-Ala ist einem natürlichen Substrat der Transglutaminase 2 entnommen (Hohenadl, C., et al., J. *Biol. Chem*. 1995,:23415-20). Durch die erfindungsgemäße Modifikation dieses Peptides kann eine kinetische Messung von Transglutaminase 2-Aktivität durchgeführt werden. Die Messung wurde in diesem Fall wie folgt durchgeführt.

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| rhHis₆TG2: | 0,25 µM |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | Exc.: 310 nm Em.: 427 nm, Messung für 20 min |

Das Ergebnis der Messung ist in Fig. 4 graphisch dargestellt.

### BEISPIEL 3

### Kinetische Messung von Meerschweinchenleberhomogenisat

Meerschweinchenleber enthält eine Transglutaminase, die in der wissenschaftlichen Literatur aufgrund ihrer starken Homologie zu humaner Transglutaminase 2 häufig als Modellenzym eingesetzt wird. Aufgrund dieser Homologie kann die Aktivität dieses Enzyms ebenfalls mit Verbindung 3 gemessen werden.

Das Homogenisat wird hergestellt nach Leblanc et al. (Leblanc et al., Protein Expr. Purif., 1999, 89-95).

Von diesem Homogenisat werden 20 µl zu 980 µl einer Lösung gegeben, so dass folgende Zusammensetzung im Ansatz vorliegt:

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| Meerschweinchenleberhomogenisat: | 20 µl |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | Exc.: 310 nm Em.: 427 nm, Messung für 20 min |

Das Ergebnis der Messung ist in Fig. 5 graphisch dargestellt.

### BEISPIEL 4

### Selektivität der Messungen verschiedener Transglutaminasen

### 1. Ansatz: Substratpeptid: Abz-Ala-Pro-Glu(CAD-DNP)-Gln-Glu-Ala (3), Transglutaminase: Faktor XIII

| | |
|---|---|
| Ansatzgröße: | 1 ml |
| Abz-Ala-Pro-Glu(CAD-DNP)-Gln-Glu-Ala (3): | 50 µM (vorgelöst in DMSO) |
| Faktor XIII: | 100 µl Citratplasma |
| Puffer: | 50 mM Tris-HCl, pH 7,5 , 10 mM CaCl₂, 100 mM NaCl, 0,1% PEG |
| DMSO-Gehalt im Ansatz: | 6 % |
| Messbedingungen: | Exc.: 310 nm Em.: 427 nm, Messung für 20 min |
| Ergebnis: kein Anstieg der Fluoreszenz | |

### 2. Ansatz: Abz-Asn-Glu(CAD-DNP)-Glu-Gln-Val-Ser-Pro-Leu-Thr-Leu-Leu-Lys (2) Transglutaminase: gereinigte rekombinante humane Transglutaminase 2

| | |
|---|---|
| Ansatzgröße: | 1 ml |
| Abz-Asn-Glu(CAD-DNP)-Glu-Gln-Val-Ser-Pro-Leu-Thr-Leu-Leu-Lys (2): | 50 µM |
| rhHis₆TG2: | 0,25 µM |
| Puffer: | 50 mM Tris-HCl, pH 7,5 , 10 mM CaCl₂, 100 mM NaCl, 0,1% PEG |
| DMSO Gehalt im Ansatz: | 6 % |
| Messbedingungen: | Exc.: 310 nm Em.: 427 nm, Messung für 20 min |
| Ergebnis: | kein Anstieg der Fluoreszenz |

## Patentansprüche

1. Verfahren zur fluoreszenzbasierten kinetischen Messung von Transglutaminase-Aktivität, **dadurch gekennzeichnet, dass** die Transglutaminase mit einem modifizierten Peptidsubstrat, bei dem ein Fluorophor direkt an den N-Terminus des Peptids gebunden ist und ein Glutaminsäurerest in 5-Position einen Fluoreszenzquencher trägt, welcher über einen Spacer, der eine Aminfunktion enthält, unter Ausbildung einer Amidbindung an die 5-Position gebunden ist, umgesetzt wird, so dass durch die Aktivität der Transglutaminase der Fluoreszenzquencher abgespalten wird, und die resultierende Fluoreszenz des Restmoleküls gemessen wird.

2. Verfahren nach Anspruch 1, **dadurch gekennzeichnet, dass** das Fluorophor ein am Benzolring substituiertes Benzoesäurederivat ist.

3. Verfahren nach Anspruch 1 oder 2, **dadurch gekennzeichnet, dass** das Fluorophor 2-Aminobenzoesäure oder N-Methyl-2-aminobenzoesäure ist.

4. Verfahren nach Anspruch 3, **dadurch gekennzeichnet, dass** das Peptidsubstrat die folgende Formel I aufweist, 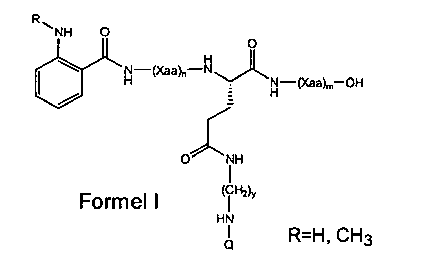
worin Xaa ein beliebige Aminosäure oder ein Aminosäurederivat davon ist,
n und und m unabhängig voneinander 0 bis 10 sind,
y 1 bis 6, vorzugsweise 5, ist,
Q ein Fluoreszenzquencher ist,
R H oder CH₃ ist.

5. Verfahren nach Anspruch 4, **dadurch gekennzeichnet, dass** das Peptidsubstrat aus der Gruppe, bestehend aus den Verbindungen Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}-NH-Q)-Gln-Ile-Val, Abz-Ala-Pro-Glu((CH₂)_{y}-NH-Q)-Gln-Glu-Ala und N-Methyl-Abz-Derivaten davon, ausgewählt ist.

6. Verfahren nach einem der Ansprüche 1-5, **dadurch gekennzeichnet, dass** der Fluoreszenzquencher eine 2,4-Dinitrophenyl- oder 3-Nitrotyrosingruppe enthält.

7. Verfahren nach einem der Ansprüche 1-6, **dadurch gekennzeichnet, dass** die Transglutaminase eine Säuger-Transglutaminase, insbesondere eine humane Transglutaminase, ist.

8. Verfahren nach Anspruch 7, **dadurch gekennzeichnet, dass** die Transglutaminase humane Transglutaminase 2 oder FXIII ist.

9. Verfahren nach einem der Ansprüche 1-8, **dadurch gekennzeichnet, dass** die Transglutaminase ein Blutgerinnungsfaktor ist und Gly-Pro-Arg-Pro-amid als Fibrinaggregationshemmer verwendet wird.

10. Verfahren nach Anspruch 8 oder 9, **dadurch gekennzeichnet, dass** die Transglutaminase Blutgerinnungsfaktor FXIII ist und das FXIII durch Thrombin aktiviert wird.

11. Verfahren nach einem der Ansprüche 1-10, **dadurch gekennzeichnet, dass** ein Amin zugesetzt wird.

12. Verfahren nach Anspruch 11, **dadurch gekennzeichnet, dass** das Amin ein Glycinmethyl- oder -ethylester, Ethanolamin, Diaminoethan oder ein Derivat von 1,5-Diaminopentan ist.

13. Verfahren nach einem der Ansprüche 1-12, **dadurch gekennzeichnet, dass** die Transglutaminase-Aktivität sowohl in gereinigter Form als auch in ungereinigter Form, z. B. in Zellhomogenisaten, gemessen werden kann.

14. Verfahren nach einem der Ansprüche 1-13, **dadurch gekennzeichnet, dass** die Messung der Aktivität einer Transglutaminase unabhängig von der Gegenwart anderer Transglutaminasen erfolgen kann.

15. Verfahren nach einem der Ansprüche 1-14, **dadurch gekennzeichnet, dass** die Aktivitätsmessung sowohl als Einzelmessung als auch als paralle Messung in einer für ein parallelisiertes Messverfahren geeigneten Vorrichtung durchgeführt werden kann.

16. Verfahren nach Anspruch 15, **dadurch gekennzeichnet, dass** die Messung in einer Mikrotiterplatte durchgeführt wird.

17. Fluoreszenzmarkiertes Peptidsubstrat für Transglutaminasen, **dadurch gekennzeichnet, dass** ein Fluorophor direkt an den N-Terminus des Peptids gebunden ist und ein Glutaminsäurerest in 5-Position einen Fluoreszenzquencher trägt, welcher über einen Spacer, der eine Aminfunktion enthält, unter Ausbildung einer Amidbindung an die 5-Position gebunden ist.

18. Peptidsubstrat nach Anspruch 17, **dadurch gekennzeichnet, dass** das Fluorophor ein am Benzolring substituiertes Benzoesäurederivat ist.

19. Peptidsubstrat nach Anspruch 18, **dadurch gekennzeichnet, dass** das Fluorophor 2-Aminobenzoesäure oder N-Methyl-2-aminobenzoesäure ist.

20. Peptidsubstrat nach Anspruch 19, **dadurch gekennzeichnet, dass** das Peptidsubstrat die folgende Formel 1 aufweist, 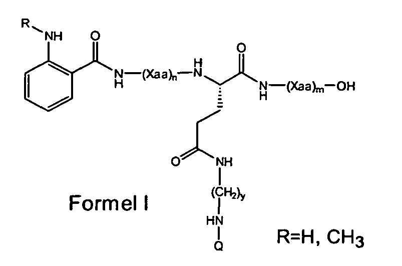
worin Xaa ein beliebige Aminosäure oder ein Aminosäurederivat davon ist,
n und und m unabhängig voneinander 0 bis 10 sind,
y 1 bis 6, vorzugsweise 5, ist,
Q ein Fluoreszenzquencher ist,
R H oder CH₃ ist.

21. Peptidsubstrat nach Anspruch 20, **dadurch gekennzeichnet, dass** das Peptidsubstrat aus der Gruppe, bestehend aus den Verbindungen Abz-Asn-Glu((CH₂)_{y}-NH-Q)-Glu-Gln-Val-Ser-Pro-Leu-Thr-Leu-Leu-Lys, Abz-Pyr-Ala-Glu((CH₂)_{y}-NH-Q)-Gln-Ile-Val, Abz-Ala-Pro-Glu((CH₂)_{y}-NH-Q)-Gln-Glu-Ala und N-Methyl-Abz-Derivaten davon, ausgewählt ist.

22. Peptidsubstrat nach einem der Ansprüche 17-21, **dadurch gekennzeichnet, dass** der Fluoreszenzquencher eine 2,4-Dinitrophenyl- oder 3-Nitrotyrosingruppe enthält.
